# EUROPEAN PATENT SPECIFICATION

(11) **EP 2 986 305 B1**
(45) Date of publication and mention of the grant of the patent: **22.11.2017**
(21) Application number: 14720264.2
(22) Date of filing: 10.04.2014
(51) Int. Cl.: A61K 35/74, A61P 39/06, A61P 17/18

(54) **THERMUS THERMOPHILUS FERMENTATION MEDIUM FOR USE IN PREVENTION AND TREATMENT OF INFRA-RED RELATED SKIN DAMAGES**
THERMOS THERMOPHILUS FERMENTATIONSMEDIUM ZUR VERWENDUNG IN DER VORBEUGUNG UND BEHANDLUNG VON INFRAROTVERMITTELTEN HAUTSCHÄDEN
MEDIUM DE FERMENTATION DE THERMUS THERMOPHILUS ET SON UTILISATION DANS LA PRÉVENTION ET LE TRAITEMENT DE LÉSIONS CUTANÉES ASSOCIÉES À UN RAYONNEMENT INFRAROUGE

(30) Priority: 16.04.2013 FR 1353420
(43) Date of publication of application: 24.02.2016
(73) Proprietor: Sederma, 78610 Le Perray en Yvelines (FR)
(72) Inventor: MONDON, Philippe, 92120 Montrouge (FR); RINGENBACH, Caroline, 78120 Rambouillet (FR); MARCHAND, Thibault, 78610 Le-Perray-En-Yvelines (FR); URSULET, Camille, 91940 Les Ulis (FR)
(86) International application number: PCT/IB2014/060608
(87) International publication number: WO 2014/170801

(56) References cited:
- WO-A1-2005/074951
- FR-A1- 2 821 086
- US-A1- 2007 237 735
- DATABASE WPI Week 200648 Thomson Scientific, London, GB; AN 2006-468688 XP002717794, & KR 2005 0040491 A (AMOREPACIFIC CORP) 3 May 2005 (2005-05-03)
- LEARY D ET AL: "Marine genetic resources: A review of scientific and commercial interest", MARINE POLICY, PERGAMON, AMSTERDAM, NL, vol. 33, no. 2, 1 March 2009 (2009-03-01), pages 183-194, XP025717161, ISSN: 0308-597X, DOI: 10.1016/J.MARPOL.2008.05.010 [retrieved on 2008-07-09]
- SCHROEDER P ET AL: "The role of near infrared radiation in photoaging of the skin", EXPERIMENTAL GERONTOLOGY, ELSEVIER SCIENCE, OXFORD, GB, vol. 43, no. 7, 1 July 2008 (2008-07-01), pages 629-632, XP022832860, ISSN: 0531-5565, DOI: 10.1016/J.EXGER.2008.04.010 [retrieved on 2008-04-27]
- DARVIN MAXIM E ET AL: "Topical beta-carotene protects against infra-red-light-induced free radicals.", EXPERIMENTAL DERMATOLOGY FEB 2011, vol. 20, no. 2, February 2011 (2011-02), pages 125-129, XP002717795, ISSN: 1600-0625
- LINTNER K ET AL: "Cosmeceuticals and active ingredients", CLINICS IN DERMATOLOGY, J.B. LIPPINCOTT, PHILADELPHIA, PA, US, vol. 27, no. 5, 1 September 2009 (2009-09-01), pages 461-468, XP026471175, ISSN: 0738-081X, DOI: 10.1016/J.CLINDERMATOL.2009.05.009 [retrieved on 2009-08-18]
- DATABASE MEDLINE [Online] US NATIONAL LIBRARY OF MEDICINE (NLM), BETHESDA, MD, US; 2009, ZASTROW L ET AL: "The missing link--light-induced (280-1,600 nm) free radical formation in human skin.", Database accession no. NLM19122479 & SKIN PHARMACOLOGY AND PHYSIOLOGY 2009, vol. 22, no. 1, 2009, pages 31-44, ISSN: 1660-5535

### SEQUENCE LISTING

<110> SEDERMA
<120> PREVENTION AND TREATMENT OF INFRA-RED RELATED SKIN DAMAGES
<130> VENIR PCT
<150> FR1353420
   <151> 2013-04-16
<160> 12
<170> PatentIn version 3.5
<210> 1
   <211> 4
   <212> PRT
   <213> artificial sequence
<220>
   <223> synthetic peptide
<400> 1
   Arg Ser Arg Lys
   1
<210> 2
   <211> 4
   <212> PRT
   <213> artificial sequence
<220>
   <223> synthetic peptide
<400> 2
   Gly Gln Pro Arg
   1
<210> 3
   <211> 4
   <212> PRT
   <213> artificial sequence
<220>
   <223> synthetic peptide
<400> 3
   Lys Thr Phe Lys
   1
<210> 4
   <211> 5
   <212> PRT
   <213> artificial sequence
<220>
   <223> synthetic peptide
<400> 4
   Lys Thr Thr Lys Ser
   1 5
<210> 5
   <211> 6
   <212> PRT
   <213> artificial sequence
<220>
   <223> Synthetic peptide
<400> 5
   Gly Lys Thr Thr Lys Ser
   1 5
<210> 6
   <211> 6
   <212> PRT
   <213> artificial sequence
<220>
   <223> synthetic peptide
<400> 6
   Val Gly Val Ala Pro Gly
   1 5
<210> 7
   <211> 4
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 7
   Gly Gln Pro Arg
   1
<210> 8
   <211> 4
   <212> PRT
   <213> artificial sequence
<220>
   <223> synthetic peptide
<220>
   <221> MOD_RES
   <222> (1)..(1)
   <223> amidation by an Elaidoyl chain on the N terminal end
<400> 8
   Lys Thr Phe Lys
   1
<210> 9
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 9
   Lys Thr Thr Lys Ser
   1               5
<210> 10
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<220>
   <221> MISC_FEATURE
   <222> (5)..(5)
   <223> Xaa being either a Methionine M or a Leucine L.
<400> 10
   Tyr Gly Gly Phe Xaa
   1               5
<210> 11
   <211> 6
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 11
   Val Gly Val Ala Pro Gly
   1               5
<210> 12
   <211> 6
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 12
   Gly Lys Thr Thr Lys Ser
   1               5

## Description

### TECHNICAL FIELD

The present invention is directed to a new product to prevent or treat damages associated with infra-red (IR) radiation of the skin and its appendages of human or animal mammals. It concerns the cosmetics, hygiene and personal care product, and dermopharmacy industries.

### BACKGROUND ART

The importance of protecting skin and appendages from sun radiation is indisputable now, but it is mostly the harmful effects of ultraviolet radiation (UVA and UVB) that are widely known: bums, blisters, inflammation, photo-ageing, photo-carcinogenesis etc. The negative effects on the skin of radiation beyond the UV, such as IR or visible radiations of very high energy (VHE) have long been ignored.

The infra-red (IR) decompose into three parts according to their wavelengths (Near-IR or IR-A: 750-1400nm, Medium-IR or IR-B 1400-3000nm and Far-IR or IR-C: 3000nm-1mm). In terms of energy, IR represent 54% of the global solar spectrum against 7% for UV. One third of the total energy of IR penetrates the skin but with differences.

Indeed 65% of IR-A deeply penetrates the dermis even reaching the hypodermis, whereas IR-B only marginally penetrates the dermis and IR-C remains in the epidermis heating it extensively due to water molecules. In recent years, a growing number of studies suggests the importance of IR in skin damage, including: increased micro-inflammatory reactions, disruption of dermal structure, oxidation, decrease of cellular energy etc. It becomes clear now that IR-A induce similar biological effects to those observed for UV but with different mechanisms: decrease of intracellular ROS and pro-inflammatory precursors, maintaining synthesis of macromolecules such as dermal collagen, stimulation of mitochondrial respiration. It therefore appears important to address also this danger to skin and its appendages.

The present invention aims to provide a solution to the problems generated in the skin and the appendages by IR radiation.

### SUMMARY OF THE INVENTION

The invention is defined by the claims. The subject matter of the present invention is a fermentation medium of *thermus thermophilus* (INCI name: "*Thermus thermophilus* Ferment" marketed by SEDERMA under the trade name VENUCEANE™) for use in preventing and/or treating damages associated with infrared radiation (IR) of the skin and its appendages. VENUCEANE™ *Thermus thermophilus* microorganisms come from marine waters located near very deep hydrothermal vents (Guaymas Basin (Gulf of California) 2000m depth, temperature 75°C, pressure 200bars). The strain used in VENUCEANE™ is the GY1211 strain (reference 2165) available from the Culture Collection of the University of Western Brittany ("Souchotèque de l'Université de Bretagne Occidendate, UBO"), LM2E collection of microorganisms from extreme environments of the Laboratory of Microbiology of Extreme Environments (IUEM).

VENUCEANE™ is an anti-aging ingredient used in cosmetic compositions for protecting the cellular structures from damages caused by UV radiation.

The inventors have shown that unexpectedly VENUCEANE™ could also provide a remarkable response with regard to damages caused by the IR radiation.

It was demonstrated in particular:
- That IR-A increase the formation of pro-inflammatory mediators (PGE2, IL-6 and IL-8) and that VENUCEANE™ can limit this increase;
- That IR-A alter dermal integrity by inducing a decrease in the synthesis of macromolecules structuring the extracellular matrix (collagen-1, fibrilin-1 and/or HSP-47 (heat shock protein)) and that VENUCEANE™ can limit this decrease;
- That IR-A increase the formation of reactive oxygen species (ROS) and that VENUCEANE™ can limit this increase and can increase ATP synthesis;
- that VENUCEANE™ improves epidermis quality (stimulation of the synthesis of involucrin, ceramides and/or intercellular lipids).

Detailed results are given below in the detailed description.

VENUCEANE™ advantageously provides a broad activity spectrum in response to the damaging effects of solar radiation, not only UV but also IR radiation.

According to other characteristics of the invention, in a cosmetic composition, the VENUCEANE™ ingredient may be combined with a sunscreen, preferably having the widest spectrum of protection, filtering UVA and UVB, but also capable of filtering most specifically IR, near-UV and/or HEV (high energy visible) radiations.

These two ingredients are complementary, the filter will physically prevent radiations to reach the skin and appendages, in particular by absorption, and the cosmetic ingredient VENUCEANE™ will prevent and/or treat the skin from harmful effects of radiations which will nevertheless reach the skin and appendages.

Suitable sunscreens may be organic or inorganic.

A wide variety of organic and inorganic conventional sunscreens can be used in the context of the present invention. The exact amount of filter varies with the chosen sunscreen and the desired Sun Protection Factor (SPF).

As examples of organic screening agents, can be listed in particular:
- Para-aminobenzoic acid (PABA) derivatives: ethyl dihydroxypropyl PABA, ethylhexyl dimethyl PABA sold in particular under the tradename ESCALOL 507™ by ISP, glyceryl PABA, PEG-25 PABA sold under the tradename UVINUL P25™ by BASF;
- Salicyclic derivatives: homosalate sold under the tradename EUSOLEX HMS™ by RONA/EM INDUSTRIES, ethylhexyl salicylate sold under the tradename NEO HELIOPAN OS™ by HAARMANN and REIMER, dipropyleneglycol salicylate sold under the tradename DIPSAL™ by SCHER, TEA salicylate sold under the tradename NEO HELIOPAN TS™ by HAARMANN and REIMER;
- Dibenzoylmethane derivatives: butyl methoxydibenzoylmethane sold in particular under the tradename PARSOL 1789™ by HOFFMANN LA ROCHE and isopropyl dibenzolylmethane;
- Cinnamic derivatives: ethylhexyl methoxycinnamate sold in particular under the tradename PARSOL MCX™ by HOFFMANN LA ROCHE, isopropyl methoxy cinnamate, isoamyl methoxy cinnamate sold under the tradename NEO HELIOPAN E 1000™ by HAARMANN and REIMER, cinoxate, DEA methoxycinnamate, diisopropyl methylcinnamate, glyceryl ethylhexanoate dimethoxycinnamate;
- ββ'-diphenylacrylate derivatives: octocrylene sold in particular under the tradename UVINUL N539™ by BASF and etocrylene sold in particular under the tradename UVINUL N35™ by BASF;
- Benzophenone derivatives: benzophenone-1 sold under the tradename UVINUL 400™ by BASF, benzophenone-2 sold under the tradename UVINUL D50™ by BASF, benzophenone-3 or oxybenzone sold under the tradename UVINUL M40™ by BASF, benzophenone-4 sold under the tradename UVINUL MS40™ by BASF, benzophenone-5, benzophenone-6 sold under the tradename HELISORB 11™ by NORQUAY, benzophenone-8 sold under the tradename SPECTRA-SORB UV-24™ by AMERICAN CYANAMID, benzophenone-9 sold under the tradename UVINUL DS-49™ by BASF and benzophenone-12;
- Benzylidene camphor derivatives: benzylidene 3-camphor, methylbenzylidene 4-camphor sold under the traname EUSOLEX 6300™ by MERCK, benzylidene camphor sulphonic acid, benzalkonium methosulphate camphor, terephthalylidene dicamphor sulphonic acid and polyacrylamidomethyl benzylidene camphor;
- Phenylbenzimidazole derivatives: phenylbenzimidazole sulphonic acid sold in particular under the tradename EUSOLEX 232™ by MERCK and benzimidazilate sold under the tradename NEO HELIOPAN AP™ by HAARMANN and REIMER,
- Triazine derivatives: anisotriazine sold under the tradename TINOSORB S™ by CIBA GEIGY, ethylhexyltriazone sold in particular under the tradename UVINUL T150™ by BASF and diethylhexyl butamido triazone sold under the tradename UVASORB HEB™ by SIGMA 3V;
- Phenylbenzotriazole derivatives: drometrizole trisiloxane sold under the tradename SILATRIZOLE™ by RHODIA CHIMIE;
- Anthranilic derivatives: menthyl anthranilate sold under the tradename NEO HELIOPAN MA™ by HAARMANN and REIMER;
- Imidazoline derivatives: ethylhexyl dimethoxybenzylidene dioxoimidazoline propionate;
- Benzalmalonate derivatives: polyorganosiloxane with benzalmalonate functional groups sold under the tradename PARSOL SLX™ by HOFFMANN LA ROCHE;
- Dihydroxycinnamic acid derivatives (umbelliferone, methylumbelliferone, methylaceto-umbelliferone); trihydroxy-cinnamic acid derivatives (esculetin, methylesculetin, daphnetin and the glucosides, esculin and daphnin); hydrocarbons (diphenylbutadiene, stilbene); dibenzalacetone and benzalacetophenone; naphtholsulfonates (sodium salts of 2-naphthol-3,6-disulfonic and of 2-naphthol-6,8-disulfonic acids); di-hydroxynaphthoic acid and its salts; o- and p-hydroxybiphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinoline derivatives (2-phenylquinoline salts); uric and violuric acids; tannic acid and its derivatives (e.g., hexaethylether); (butyl carbotol) (6-propyl piperonyl) ether; hydroquinone;

The organic UV-screening agents which are more particularly preferred are chosen from the following compounds: ethylhexyl salicylate, butyl methoxydibenzoylmethane, ethylhexyl methoxycinnamate, octocrylene, phenylbenzimidazole sulphonic acid, terephthalylidene dicamphor sulphonic, benzophenone-3, benzophenone-4, benzophenone-5, 4-methylbenzylidene camphor, benzimidazilate, anisotriazine, ethylhexyl triazone, diethylhexyl butamido triazone, methylene bis-benzotriazolyl tetramethylbutylphenol, drometrizole trisiloxane, and mixtures thereof.

Also preferred are the compositions described in U.S. Patent No. 6,190,645 and in particular, sunscreen agents sold under the tradename INCROQUAT-UV-283™ manufactured by CRODA.

The inorganic screening agents which may be used in the composition according to the invention are in particular nanopigments (mean size of the primary particles: generally between 5 nm and 100 nm, preferably between 10 nm and 50 nm) of coated or uncoated metal oxides such as for example nanopigments of titanium oxide (amorphous or crystallized in the form of rutile and/or anatase), iron, zinc, zirconium, cerium or manganese oxides. Coating agents are moreover alumina and/or aluminum stearate. Such nanopigments of metal oxides, coated or uncoated, are in particular described in EP0518772 and EP0518773.

As a preferred example, the inorganic filter sold by Croda under the range commercial tradename SOLAVEIL™ can be advantageously mentioned, having a broad spectrum of protection, especially remarkable with regard to UVA and/or UVB radiations.

Another prefered inorganic sunscreen is OPTISOL™ sold by OXONICA or a manganese modified TiO2 filter (TiO2Mn) The TiO2M provides to the skin advantgeously in addition to its filtering action with regard to UV, an unifying, matting and brightening effect without white residue.

Actives known to protect and/or treat damages to the skin or its appendages caused by UV, can be advantageously used in reinforcement of activity, including the following commercial actives: Cosmedia DC™ of Cognis-Care Chemicals, Helioguard 365™, NanoVit™ and PhytoCellTec™ Solar Vitis of Mibelle AG Cosmetics, Melaneze™ of Dragoco, Vanirea™ of Solabia, Solarine III™, Nucleolys™ and Sun Protection complex™ of Greentech, Pronalen Sunlife™ of S. Black Ltd., ClC2™ and Antikeuline 6™ of Biotech Marine (Secma Biotechnologies Marines), Parsol SLX™ and Alpaflor Edelweiss™ of DSM Nutritional Products, DN-AGE2™, DN-AGE LS 9547™ and Sunactyl LS 9610™ of Laboratoires Sérobiologiques, Uniprotect PT 3™ d'Induchem, Maricol S CLR™ of Chemisches Laboratorium Dr. Richter, Illumiscin™ of Indena, Caspaline 14™ and Natriance Antioxidizer™ of Vincience/ISP, Liposhield HEV Melanin™ of Lipo Chemicals, Fruit Vinegar™ of Provital SA, IBR-TCLC 0701™ of IBR and Muciliance Fruit™ of Soliance.

### DETAILED DESCRIPTION

The present invention will be better understood and other advantages will become apparent from the following detailed description.

### A) Preparation of compositions that can be used according to the invention

A cosmetic composition that can be used in the context of the present invention will comprise an amount of *thermus thermophilus* fermentation medium (a percentage of the VENUCEANE™ active) in a physiologically acceptable medium.

"Physiologically acceptable medium" means according to the present invention, without limitation, an aqueous or hydroalcoholic solution, a water-in-oil emulsion, an oil-in-water emulsion, a microemulsion, an aqueous gel, an anhydrous gel, a serum, a dispersion of vesicles, or a powder.

"Physiologically acceptable" means that the compositions are suitable for topical or oral use, in contact with mucous membranes, appendages (nails, hair and body hair), scalp and skin of mammals, particularly human, compositions which may be ingested or injected into the skin, without risk of toxicity, incompatibility, instability, allergic response, and others.

The composition according to the invention can be applied to the face, body, neck, neckline, scalp, hair, eyelashes, body hair, in any form or vehicles known from the ones skilled in the art, in particular in the form of solution, dispersion, emulsion, paste or powder, individually or as a premix in vectors such as macrocapsules, microcapsules or nanocapsules, macrospheres, microspheres or nanospheres, liposomes, oleosomes or chylomicrons, macroparticules, microparticules or nanoparticules, macrosponges, microsponges or nanosponges, microemulsions or nanoemulsions, or adsorbed on organic polymer powders, talcs, bentonites, spores or exines and other inorganic or organic supports.

In cosmetics for example, applications can be proposed in particular in the ranges of skin care products for face, body, hair and body hair and makeup-care lines, including eyebrows and eyelashes, in particular anti-aging lines, and sun protective and after sun lines.

The fermentation medium of *thermus thermophilus* VENUCEANE™ of the present invention may in general be used in any form whatsoever, in a form bound to or incorporated in or absorbed in or adsorbed on macro-, micro-, and nanoparticles, or macro-, micro-, and nanocapsules, for the treatment of textiles, natural or synthetic fibers, wools, and any materials that may be used for clothing or underwear for day or night intended to come into contact with the skin, handkerchiefs or cloths, to exert their cosmetic or therapeutical effect via this skin/textile contact and to permit continuous topical delivery.

As explained above, the fermentation medium of *thermus thermophilus* VENUCEANE™ can be combined with other ingredients active at effective concentrations that can act synergistically or to reinforce the effect to achieve the desired effects described for the invention, such as the following agents: radiation filters, including UVA and/or UVB as seen above, hydrating, calming, muscle relaxant, slimming, restructuring, firming, plumping, tensor, acting on microcirculation, on inflammation, on free radicals, vitamins, anti-wrinkle, whitening agents, etc.

The CTFA International cosmetic ingredient dictionary & handbook (13th Ed. 2010) (published by the Cosmetic, Toiletry, and Fragrance Combination, Inc., Washington, D.C.) describes a non-limited wide variety of cosmetic and pharmaceutical ingredients conventionally used in the skin care industry that can be used as additional ingredients/compounds in the compositions of the present invention.

Further skin care and hair care active ingredients that are particularly useful can be found in Sederma's commercial literature and on the website www.sederma.fr.

The following commercial actives can also be mentioned, as examples: betain, glycerol, Actimoist Bio 2™ (Active organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals, Inc), Hydra'Flow™ (Sochibo), Hydromoist L™ (Symrise), RenovHyal™ (Soliance), Seamoss™ (Biotech Marine), Argireline™ (trade name of the acetyl hexapeptide-3 of Lipotec), spilanthol or an extract of *Acmella oleracea* known under the name Gatuline Expression™ an extract of *Boswellia serrata* known under the name Boswellin™, Deepaline PVB™ (Seppic), Syn-AKE™ (Pentapharm), Ameliox™, Bioxilift™ (Silab), PhytoCellTec™Argan (Mibelle), Papilactyl D™ (Silab), Preventhelia™ (Lipotec), Subliskin™ (Sederma), Moist 24™ (Sederma), Vegesome Moist 24™ (Sederma), Essenskin™ (Sederma), Juvinity™ (Sederma), Revidrate™ (Sederma), Resistem™ (Sederma), Chronodyn™ (Sederma), Kombuchka™ (Sederma), Chromocare™ (Sederma), Calmosensine™ (Sederma), Glycokin factor S™ (Sederma), Biobustyl™ (Sederma), Idealift™ (Sederma), Ceramide 2™, Ceramide A2™ and Ceramide HO3™ (Sederma), Legance™ (Sederma), Intenslim™ (Sederma), Prodizia™ (Sederma), Beautifeye™ (Sederma), Senestem™ (Sederma) or mixtures thereof.

Among the plant extracts which can be combined with the *thermus thermophilus* ferment of the invention, there may more particularly be mentioned extracts of Ivy, in particular English Ivy (*Hedera Helix*), of *Bupleurum chinensis,* of *Bupleurum Falcatum,* of arnica (*Arnica Montana L*), of rosemary (*Rosmarinus officinalis N*), of marigold (*Calendula officinalis*), of sage (*Salvia officinalis L*), of ginseng (*Panax ginseng*), of ginko biloba, of St.-John's-Wort (*Hyperycum Perforatum*), of butcher's-broom (*Ruscus aculeatus L*), of European meadowsweet (*Filipendula ulmaria L*), of big-flowered Jarva tea (*Orthosiphon Stamincus Benth*), of algae (*Pucus Vesiculosus*), of birch (*Betula alba*), of green tea, of cola nuts (*Cola Nipida*), of horse-chestnut, of bamboo, of *Centella asiatica,* of heather, of fucus, of willow, of mouse-ear, of escine, of cangzhu, of *chrysanthellum indicum,* of the plants of the *Armeniacea* genus, *Atractylodis Platicodon, Sinnomenum, Pharbitidis, Flemingia,* of *Coleus* such as *C*. *Forskohlii, C*. *blumei, C*. *esquirolii, C*. *scutellaroides, C*. *xanthantus* and *C*. *Barbatus,* such as the extract of root of *Coleus barbatus,* extracts of *Ballote,* of *Guioa,* of *Davallia,* of *Terminalia,* of *Barringtonia,* of *Trema,* of *antirobia, cecropia, argania, dioscoreae* such as *Dioscorea opposita* or Mexican, extracts of *Ammi visnaga,* of *Siegesbeckia,* in particular *Siegesbeckia orientalis,* vegetable extracts of the family of *Ericaceae,* in particular bilberry extracts (*Vaccinium angustifollium*) or *Arctostaphylos uva ursi, aloe vera,* plant containing sterols (e.g., phytosterol), Manjistha (extracted from plants of the genus *Rubia,* particularly *Rubia Cordifolia*), and Guggal (extracted from plants of the genus *Commiphora,* particularly *Commiphora Mukul*), kola extract, chamomile, red clover extract, Piper methysticum extract (Kava Kava™ from SEDERMA), *Bacopa monieri* extract (Bacocalmine™ from SEDERMA) and sea whip extract, extracts of *Glycyrrhiza glabra,* of mulberry, of *melaleuca* (tea tree), of *Larrea divaricata,* of *Rabdosia rubescens,* of *Euglena gracilis,* of *Pibraurea recisa Hirudinea,* of *Chaparral Sorghum,* of sun flower extract, of *Enantia chlorantha,* of Mitracarpe of *Spermacocea* genus, of *Buchu barosma,* of *Lawsonia inermis L.,* of *Adiantium Capillus-Veneris L.,* of *Chelidonium majus,* of *Luffa cylindrica,* of Japanese Mandarin (*Citrus reticulata Blanco* var. *unshiu*), of *Camelia sinensis,* of *Imperata cylindrica,* of *Glaucium Flavum,* of *Cupressus Sempervirens,* of *Polygonatum multiflorum,* of *loveyly hemsleya,* of *Sambucus Nigra,* of *Phaseolus lunatus,* of *Centaurium,* of *Macrocystis Pyrifera,* of *Turnera Diffusa,* of *Anemarrhena asphodeloides,* of *Portulaca pilosa,* of *Humulus lupulus,* of *Coffea Arabica,* of *Ilex Paraguariensis,* of *Globularia Cordifolia,* of *Albizzia julibrissin, Oxydendron arboretum* or of *Zingimber Zerumbet Smith.*

The compositions of the present invention may include peptides, including, without limitation, the di-, tri-, tetra-, penta-and hexapeptides and their derivatives. According to a particular embodiment, the concentration of the additional peptide, in the composition, ranges from 1x10⁻⁷% and 20%, preferably from 1x10⁻⁶% and 10%, preferably between 1x10⁻⁵% and 5%, by weight.

According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides which are found in nature, and/or are commercially available.

Suitable dipeptides for use within the scope of the present invention include but are not limited to carnosine (beta-AH), YR, VW, NF, DF, KT, KC, CK, KP, KK or TT. Non limitative suitable tripeptides for use herein include, but are not limited to RKR, HGG, GHK, GKH, GGH, GHG, KFK, KPK, KMOK, KMO₂K or KAvaK. Non limitative suitable tetrapeptides for use herein include but are not limited to RSRK (SEQ ID NO: 1), GQPR (SEQ ID NO: 2) or KTFK (SEQ ID NO: 3). Non limitative suitable pentapeptides include, but are not limited to KTTKS (SEQ ID NO: 4) and hexapeptides include but are not limited to GKTTKS (SEQ ID NO: 5) and VGVAPG (SEQ ID NO: 6).

Other suitable peptides for use in the context of the present invention include, but are not limited to: lipophilic derivatives of peptides, preferably palmitoyl derivatives, and metal complexes as aforementioned (e.g. copper complex of the tripeptide HGG). Preferred dipeptide derivatives include N-Palmitoyl-beta-Ala-His, N-Acetyl-Tyr-Arg-hexadecylester (Calmosensine™, Idealift™ from Sederma). Preferred tripeptide derivatives include in particular the N-Palmitoyl-Gly-Lys-His, and Pal-Gly-His-Ly, (Pal-GKH and Pal-GHK from Sederma), the copper derivative of HGG (Lamin™ from Sigma), Lipospondin (N-Elaidoyl-KFK) and its analogues of conservative substitution, N-Acetyl-RKR-NH₂ (Peptide CK+), N-Biot-GHK (from Sederma), Pal-KMO₂K (Sederma) and derivatives thereof. Suitable tetrapeptide derivatives for use according to the present invention include, but are not limited to, N-palmitoyl-GQPR (SEQ ID NO: 7) (from Sederma), Ela-KTFK (SEQ ID NO: 8). Suitable pentapeptide derivatives for use herein include, but are not limited to, N-Palmitoyl-KTTKS (SEQ ID NO: 9) (available as Matrixyl™ from Sederma), N-Palmitoyl-Tyr-Gly-Gly-Phe-X (SEQ ID NO: 10) with X Met or Leu or mixtures thereof. Suitable hexapeptide derivatives for use herein include, but are not limited to, N-Palmitoyl-VGVAPG (SEQ ID NO: 11), Pal-GKTTKS (SEQ ID NO: 12) and derivatives thereof. The mixture of Pal-GHK and Pal-GQPR (SEQ ID NO: 7) (Matrixyl™ 3000, Sederma) can also be mentioned.

The preferred compositions commercially available containing a tripeptide or a derivative include Biopeptide-CL™, Maxilip™, Biobustyl™, Procapil™ and Matrixyl™synthe'6™ of Sederma. The compositions commercially available preferred sources of tetrapeptides include Rigin™, Eyeliss™, Matrixyl™ Reloaded and Matrixyl 3000™ which contain between 50 and 500 ppm of Palmitoyl-GQPR (SEQ ID NO: 7) and carrier, proposed by Sederma.

The following marketed peptides can be mentioned as well as additional active ingredients: Vialox™, Syn-ake™ or Syn-Coll™ (Pentapharm), Hydroxyprolisilane CN™ (Exsymol), Argireline™, Leuphasyl™, Aldenine™, Trylgen™, Eyeseryl™, Serilesine™ or Decorinyl™ (Lipotec), Collaxyl™ or Quintescine™ (Vincience), BONT-L-Peptide™ (lnfinitec Activos), Cytokinol™LS (Laboratoires Serobiologiques/Cognis), Kollaren™, IP2000™ or Meliprene™ (lnstitut Européen de Biologie Cellulaire), Neutrazen™ (Innovations), ECM-Protect™ (Atrium Innovations), Timp-Peptide™ or ECM Moduline™ (lnfinitec Activos).

More specifically, the *Thermus thermophilus* ferment may be combined with at least one of the compounds selected from vitamin B3 compounds, such as tocopherol or niacinamide, retinoid compounds such as retinol, hexamidine, α-lipoic acid, resveratrol or DHEA, peptides, in particular N-acetyl-Tyr-Arg-O-hexadecyl, Pal-VGVAPG (SEQ ID NO: 11), Pal-KTTKS (SEQ ID NO: 12) Pal-GHK, Pal-KMO2K and Pal-GQPR (SEQ ID NO: 7), which are conventionally used active ingredients in topical cosmetic or dermopharmaceutical compositions.

The *thermus thermophilus* fermentation medium according to the invention may be applied locally to targeted areas.

The "effective" amount depends on various factors, such as the age, the phenotype and condition of the patient, the severity of the disorder or disease and the administration mode. An effective amount means a non-toxic amount enough to achieve the desired effect.

In a cosmetic composition according to the invention, the fermentation medium to be present in an effective amount, is usually present in an amount ranging from 0.000001% and 15% based on the total weight of the composition, preferably between 0.0001% and 10% depending on the destination of the composition and the more or less pronounced desired effect.

All percentages and ratios used herein are by weight of the total composition and all measurements are made at 25°C unless it is otherwise specified.

For example, for a face cosmetic treatment, the SCCS'S (Scientific Committee on Consumer Safety) Notes of Guidance for the testing of cosmetic substances and their safety evaluation (8th Revision, 11 dec. 2012) has set a standard amount for applying a cream of 2.72 mg/cm2/day/person and for a body lotion of 0.5 mg/cm2/day/person.

According to other specific features, the cosmetic treatment method according to the invention can be combined with one or more other treatment methods targeting the skin such as lumino-therapy, heat or aromatherapy treatments.

According to the invention, devices with several compartments or kits may be proposed to apply the method described above which may include for example and non-restrictively, a first compartment containing a composition comprising VENUCEANE™, and in a second compartment an excipient and/or an additional active such as a sunscreen, the compositions contained in the said first and second compartments in this case being considered to be a combination composition for simultaneous, separate or stepwise use in time, particularly in one of the treatments recited above.

### B) In vitro Evaluations

### 1) Effect on pro-inflammatory mediators

IR-A induce the formation and release of pro-inflammatory messengers. IL-6 induces premature aging and senescence in human fibroblasts as well as PGE2 compounds also critical for the induction of senescence and maintenance of an inflammatory condition.

Human dermal fibroblasts (HDF) were irradiated with an IR-lamp. Cells were contacted with VENUCEANE™ 24h before irradiation and 24 hours after, but not during. The amounts of pro-inflammatory species produced by the cells after the last contact with VENUCEANE™ were measured and compared with the results of the negative control. Exposure temperatures were controlled and remained close to the irradiated cases or not.

**Table 1: Variation of the amount of PGE2, IL-6 and IL-8 produced by HDF in contact with VENUCEANE™ (Eq1.5%) (n=3)**

| **Variation** (%) | **PGE₂** | | **IL-6** | | **IL-8** | |
|---|---|---|---|---|---|---|
| Control | Ref 1 | - | Ref 1 | - | Ref 1 | - |
| IR-A | **+31%;** *p*<*0.01* | Ref 2 | **+31%;** *p*<*0.01* | Ref 2 | **+18%;** *p*<*0.01* | Ref 2 |
| **VENUCEANE**™ + IR-A | - | **-54%;** *p*<*0.01* | - | **-53%;** *p*<*0.01* | - | **-61%;** *p*<*0.01* |

The above results indicate that IR-A radiation causes the expected increase in the formation of pre-inflammatory mediators and that VENUCEANE™ can control these increases.

### 2) Effect on the macromolecules structuring the dermis

### 2.1. Collagen

IR-A alter the integrity of the extracellular matrix of the dermis.

HDF were contacted with VENUCEANE™ for 24 hours and irradiated with IR-A in the absence of VENUCEANE™, then the cells were again contacted with VENUCEANE™ for 24 hours. A second full-length sequence was then realized, and then after 4 days of culture without VENUCEANE™, deposited collagen-1 quantification was made on photographs after immunofluorescence staining of collagen. Cell number was estimated using a marking of nuclei with Hoechst 33258 dye.

**Table 2: Change in the amount of collagen-1 produced by HDF on contact with VENUCEANE™ (n=27photos/case)**

| **Collagen-1** | **Variation (%)** | |
|---|---|---|
| Control | Reference 1 | - |
| IR-A | -**80**.**1%;** *p<0*.*01* | Reference 2 |
| **VENUCEANE™** *(Eq 1.5%)* + IR-A | - | +**81.3%**; *p*<*0.01* |

These results show the depressant effect of IR-A on the synthesis of collagen-1 and the protective effect of VENUCEANE™ with regard to these radiations.

### 2.2. HSP-47 protein (Heat Shock Protein)

HSP-47 is a protein of the dermal extracellular matrix coordinating maturation and organization, and therefore the functionality of collagen. It is a true collagen chaperone molecule.

HSP-47 declines with age, its absence leading to lower synthesis of collagens I and IV. In mice this induces a weakened skin and a weakening of the dermo-epidermal junction, their collagen being more sensitive to proteases and heat. To the contrary, its experimental overexpression in mice increases the secretions of collagen I.

Variations in the HSP-47 synthesis under IR-A stress and modulation obtained by VENUCEANE™ were studied. IR-A irradiated HDF received VENUCEANE™ before and after this irradiation. The cells were crushed and HSP47 evaluated by Western Blot after extraction and immuno-preparation. A same amount of protein was deposited.

**Table 3: Variation in the HSP-47 produced by HDF under IR-A stress +/- VENUCEANE™**

| **HSP-47** | **Variation (%)** | |
|---|---|---|
| Control | Reference 1 | - |
| IR-A | **-22%** | Reference 2 |
| **VENUCEANE™** *(eq 1.5%)* + IR-A | - | **+37.7%** |

The results indicate that IR-A cause the decrease of the synthesis of HSP-47 (-22% *vs.* control); to the contrary VENUCEANE™ can limit the decline of HSP-47 of 37.7%.

### 2.3. Fibrillin-1

Besides the two proteins of interest for dermal quality studied above, fibrillin-1 was also investigated. Fibrillin-1 is the main component of the microfibrils. It is a glycoprotein, architect of dermis, enabling the organization of the elastic fibers. It is also a fragile protein, real skin marker of photo-aging due to UV. Its decrease usually leads to a reduction of the elastic properties of the skin. Changes in its synthesis by HDF under repeated IR-A stress were monitored.

The results indicate that IR-A causes the decrease of fibrillin-1 synthesis and that VENUCEANE™ can limit this decrease.

Thus these studies realized on three macromolecules important for the quality of the dermis, collagen-1, HSP-47 and fibrillin-1, indicate that VENUCEANE™ thanks to its properties is able to fight effectively against the harmful effects of IR-A.

### 3) Effects on reactive oxygen species (ROS) and ATP

### 3.1. ROS

IR-A induce the production of radical oxygen species (or ROS) in the cells. This formation, although more limited than that formation caused by UV radiation, for example of solar origin, reinforces the deleterious effects of the latter. It leads in particular to lower capacity of ATP synthesis in mitochondria. The effects of IR-A on intracellular ROS production were studied and the effects of VENUCEANE™ in this model observed. For this, human keratinocytes were exposed to a IR-A stress and then were contacted with VENUCEANE™ for 24 h. This sequence was repeated and the cells were labeled with a chlorinated derivative of fluorescein in order to estimate the amount of ROS.

**Table 4: Variation of the intracellular ROS produced by IR-A stressed keratinocytes +/-VENUCEANE™**

| **ROS** | **Variation (%)** | |
|---|---|---|
| Control | Reference 1 | - |
| IR-A | **+29%**; *p*<*0.01* | Reference 2 |
| **VENUCEANE™** *(eq 1.5%)* + IR-A | - | **-28%;** *p*<*0.01* |

### 3.2. ATP

The capacity of VENUCEANE™ to stimulate ATP production was evaluated on human keratinocytes which ATP synthesis was reduced experimentally beforehand. For this, the cells were emptied of ATP and then received VENUCEANE™. The obtained ATP amount was compared with the negative control.

**Table 5: Variation in the amount of ATP produced by keratinocytes +/- VENUCEANE™ (n=3)**

| **ATP** | **Variation (%)** |
|---|---|
| Control | Reference 2 |
| **VENUCEANE™** *(eq 1.5%)* | +**123%;** *p*<*0.01* |

These data indicate therefore that VENUCEANE™ helps counter the formation of intracellular ROS known to cause alterations in the energy machinery of mitochondria. In parallel VENUCEANE™ stimulates ATP neosynthesis in keratinocytes (but also in fibroblasts) and can thus meet the energy needs of the cell.

### 4) Improvement of epidermis qualities

### 4.1. Involucrin

The skin has several layers that insulate and protect the rest of the body. These layers are formed from basal keratinocytes that give daughter cells. The latters transform and strengthen themselves by producing around them a shell very resistant to exterior attacks. This shell is composed of proteins strongly bonded to each other such as involucrin.

Human keratinocytes in culture contacted with VENUCEANE™ were studied in terms of the stimulation mechanisms reinforcing epidermis evaluated on standardized photographs by following the increase of labeled involucrin using a specific fluorescent antibody. The results showed a clear appearance of this protein marker in the studied cultures (x 57; *p<0.01*)*,* compared to low differentiated controls, reflecting the stimulating effect of VENUCEANE™ on these cells in the development of reinforcing structures of the epidermis.

### 4.2. Ceramides

In addition to the shell of proteins strongly bonded to each other, the epidermis in differentiation produces itself an extracellular matrix of lipids forming a peripheral sealing skin envelope. Among these the most known lipids are ceramides, which are very complex lipids structuring the cornified layer.

Cultured human keratinocytes were contacted with VENUCEANE™ and the stimulation of the formation of the lipidic envelope was assessed by following the increase of ceramide sphingosinic precursors, these precursors increasing with keratinocyte differentiation.

**Table 6: Variation in the ceramide precursor formation by keratinocytes +/- VENUCEANE™ (n=15).**

| **Ceramide precursor** | **Variation (%)** |
|---|---|
| Control | Reference 1 |
| **VENUCEANE™** *(Eq 3%)* | +**158%**; *p<0.01* |

### 4.3. Intercellular lipids

Other lipids are formed during differentiation and they accompany the formation of the lipidic envelope. They have a supporting and organization role of other lipids and proteins of the corneocyte. They also control the lipophilic and hydrophilic characteristics of these areas and the activities of enzymes present between the corneocytes. These uncharged lipids can be highlighted with a colorimetric labeling. Image analysis of the realized photographs made it possible to quantify the deposits and to compare cases with or without VENUCEANE™.

**Table 7: Variation in the neutral lipid formation by keratinocytes +/ VENUCEANE™ (n=15)**

| **Lipids** | **Variation (%)** |
|---|---|
| Control | Reference 1 |
| **VENUCEANE™** *(Eq 1%)* | **X 5.8 times;** *p*<*0.01* |

### C) Galenic

Different cosmetic formulations are described below. Additional cosmetic active ingredients, in support and/or in complement of the activity of the active ingredient according to the invention if necessary, can be added to the appropriate phase according to their hydrophobic or hydrophilic nature. These ingredients can be of any class according to their(s) function(s), place of application (body, face, neck, chest, hands, hair, eyelashes, eyebrows, body hair, etc..), final desired effect and target consumer, for example anti-aging, anti-wrinkle, moisturizing, against dark circles under the eyes, firming, anti-glycation, slimming, soothing, myo-relaxing, anti-redness, anti-stretch marks, etc. Anti-IR active ingredient according to the invention: **VENUCEANE**™ [*Thermus Thermophilus* Ferment & Glycerin].

### 1) Cream form

| **Ingredients** | **Weight %** |
|---|---|
| ***Phase A*** | |
| Crodamol CAP [Cetearyl Ethylhexanoate) | 5.00 |
| Crodafos CES [Cetearyl Alcohol & Dicetyl Phosphate & Ceteth-10 Phosphate] | 4.00 |
| Span 60 [Sorbitan Stearate] | 0.50 |
| Crodamol AB [C12-C15 Alkyl Benzoate] | 2.00 |
| Crodamol GTCC [Caprylic/Capric Triglyceride] | 4.00 |

| ***Phase B*** | |
|---|---|
| Potassium sorbate | 0.20 |
| H₂O | Qsp 100 |

| ***Phase C*** | |
|---|---|
| Glycerin | 3.00 |
| Panstat [Methyl & Ethyl & Propyl parabens] | qs |

| ***Phase D*** | |
|---|---|
| Sodium hydroxyde 30% | 0.20 |
| H₂O | 2.00 |

| ***Phase E*** | |
|---|---|
| **VENUCEANE™** [*Thermus Thermophilus* Ferment & Glycerin] | 5.00 |

Protocol: weigh phase A and heat at 75°C using a water bath. Weigh phase B. Heat phase C until dissolved, and then add it to phase B. Heat phase B+C at 75°C using a water bath. Pour phase A into phase B+C under stirring. Extemporaneously adjust the pH with phase D. Homogenize thoroughly. Then add phase E around 35°C and mix well. Check the pH around 6.00 otherwise adjust with phase D. Mix thoroughly.

### Examples of ingredients that can be added to this formulation:

CALMOSENSINE™: soothing active for sensitive skins marketed by Sederma (two1998/07744) comprising the Tyr-Arg lipo-dipeptide. It reduces discomfort feelings.

MATRIXYL3000™: peptide-based anti-wrinkle ingredient marketed by Sederma (WO2005/048968) comprising two matrikines Pal-GHK and Pal-GQPR, which in synergy helps repairing skin damages caused by aging. 3% by weight can be added for example in phase E of the formula.

MATRIXYL synthe'6™: peptide-based anti-wrinkle ingredient marketed by Sederma which helps repair skin damage caused by aging.

SENESTEM™: anti-aging active ingredient marketed by Sederma comprising meristematic cells of *Plantago lanceolata.*

### 2) Serum form

| **Ingredients** | **Weight %** |
|---|---|
| ***Phase A*** | |
| H₂O | Qsp 100 |
| ViscOptima PL [Sodium Polyacrylate (and) Paraffinum Liquidum (and) Trideceth-6] | 0.35 |

| ***Phase B*** | |
|---|---|
| Cyclopentasiloxane (and) Cyclohexasiloxane | 2.00 |
| Crodamol AB-LQ-(RB) [C12-15 Alkyl Benzoate] | 4.50 |
| Cromollient DP3A-LQ-(MH) [Di-PPG-3 Myristyl Ether Adipate] | 1.00 |
| Crodamol GTEH-LQ-(MV) [Triethylhexanoin] | 2.00 |
| Crodamol CSO-LQ-(JP) [Ceteraryl Ethylhexanoate] | 3.00 |
| Optasense G82 [Acrylates/C10-30 Alkyl Acrylate Crosspolymer] | 0.15 |

| ***Phase C*** | |
|---|---|
| Glycerin | 5.00 |
| Phenoxyethanol | Qs |

| ***Phase D*** | |
|---|---|
| Potassium sorbate | 0.10 |

| ***Phase E*** | |
|---|---|
| H₂O | 1.30 |
| Sodium hydroxyde 30% | 0.13 |

| ***Phase F*** | |
|---|---|
| **VENUCEANE™** [*Thermus Thermophilus* Ferment & Glycerin] | 5.00 |

| ***Phase G*** | |
|---|---|
| Perfume | 0.10 |

Protocol: Disperse phase A in water under stirring. Weigh and mix phase B. Weigh and mix phase C. Weigh D. Add phase B to phase A under vigorous stirring phase. Extemporaneously add phase C and phase D to phase A+C. Neutralize with phase E and stir for 30 minutes. Add phase F, mix well. Add phase G, mix well. Check the pH to 6.00 +/- 0.10.

### Examples of ingredients that can be added to this formulation:

CHRONODYN™: active ingredient marketed by SEDERMA (WO2006/075311), which tones and firms the skin, erases signs of fatigue.

RENOVAGE™: global anti-aging active ingredient marketed by SEDERMA (WO2006/120646). MELASLOW™: active ingredient marketed by Sederma that promotes lightening of skin tone and depigmentation of spots (extract of Japanese Mandarin *reticulata Blanco* var. *unshiu Citrus*).

### 3) Day cream form comprising an inorganic sunscren (SOLAVEIL™)

| **Ingredients** | **Weight %** |
|---|---|
| ***Phase A*** | |
| H₂O | Qsp 100 |

| ***Phase B*** | |
|---|---|
| Magnesium Aluminium Silicate | 0.80 |
| Xanthan Gum | 0.70 |

| ***Phase C*** | |
|---|---|
| Arlacel 2121-FL-(MV) [Sucrose Cocoate (&) Sorbitan stearate] | 2.50 |
| Crodacol S95-PA-(RB) [Stearyl Alcohol] | 2.00 |
| Tween 60-LQ-(MV) [Polysorbate 60] | 2.00 |
| Crodamol IPIS-LQ-(MV) [Isopropyl Isostearate] | 5.00 |
| Crodamol GTEH-LQ-(MV) [Triethylhexanoin] | 5.00 |
| Crodamol HD-LQ-(RB) [Isohexadecane] | 7.50 |

| ***Phase D*** | |
|---|---|
| **SOLAVEIL** CT-300-LQ-(WD) [Caprylic/Capric Triglyceride (and) Titanium | 8.00 |
| Dioxide (and) Polyhydroxystearic Acid (and) Aluminium Stearate (and) Alumina] | |
| **SOLAVEIL** CZ-300-LQ-(WD) [Zinc Oxide (and) Caprylic/Capric Triglyceride (and) Polyhydroxystearic Acid (and) Isostearic Acid] | 4.00 |

| ***Phase E*** | |
|---|---|
| Butylen glycol | 4.00 |
| Phenoxyethanol | qs |

| ***Phase F*** | |
|---|---|
| H₂O | 6.00 |
| Lactic acid | 0.60 |

| ***Phase G*** | |
|---|---|
| **VENUCEANE™** *[Thermus Thermophilus* Ferment & Glycerin] | 5.00 |

| ***Part H*** | |
|---|---|
| Perfume | 0.10 |

Protocol: Weigh phase A. Weigh phase B and gradually sprinkle in phase A under stirring until completely dissolved. Heat phase A+B at 80°C using a water bath. Weigh and heat phase C at 80°C in a water bath. Mix well. Mix phase C with phase A+B under stirring. Keep stirring for 1 hour in a water bath at 80°C. Weigh and heat phase D at 80°C in a water bath. Pour phase D into phase A+B+ C while stirring outside the bath. Weigh and add phase E in the previous phase under stirring. Add phase F under stirring and homogenize for 1 hour. Add phase G, mix well. Add phase H.

### Examples of ingredients that can be added to this formulation:

LUMISHERE™: active ingredient marketed by Sederma (WO2004/024695). It is the combination of diacetylboldine (DAB) encapsulated in polymethylmethacrylate microcapsules and titanium dioxide modified with manganese (TiO₂Mn). The TiO₂Mn gives the skin a unifying, mattifying and luminous effect and DAB provides a physiological lightening effect.

MEIRITAGE™: anti-ageing active ingredient based on three plant extracts (*Astragalus membranaceus* (Huang Qi), *Bupleurum falcatum* (Chai Hu) and *Atractylodes macrocephala* (Bai Zhu), which improves the uniformity and radiance of skin.

EYELISS™: active ingredient marketed by Sederma (WO2003/068141) that helps prevent against the appearance of bags under the eyes. It combines three components: hesperidin methyl chalcone reducing capillary permeability, Valyl-Tryptophan (VW) dipeptide which promotes lymphatic circulation and Pal-GQPR lipopeptide that improves firmness, elasticity and reduces inflammation. 3% of this ingredient can be for example added to phase E of the formulation.

SUBLISKIN™: active ingredient marketed by SEDERMA (WO2010/067327) that moisturizes and smooths the skin while allowing it to resist to external aggressions.

### 4) Day cream form comprising an organic sunscren (EUSOLEX™)

| **Ingredients** | **Weight %** |
|---|---|
| ***Phase A*** | |
| H₂O | Qsp 100 |
| Potassium sorbate | 0.10 |

| ***Phase B*** | |
|---|---|
| Butylen glycol | 6.00 |
| Phenoxyethanol | qs |
| Hydroxypropyl Guar | 0.40 |

| ***Phase C*** | |
|---|---|
| Crodafos MCA-SO-(RB) [Cetyl Phosphate] | 2.00 |
| Brij S2-SS-(RB) [Steareth-2] | 1.00 |
| Arlacel 170-PA-(RB) [Glyceryl Stearate (&) PEG-100 stearate] | 3.00 |
| Stearic Acid | 2.50 |
| EUSOLEX 4360 [Benzophenone-3] | 1.80 |
| EUSOLEX 2292 [Ethylhexyl Methoxycinnamate (and) BHT] | 3.50 |
| Cyclopentasiloxane (and) Cyclohexasiloxane | 3.00 |
| Crodamol AB-LQ-(RB) [C12-15 Alkyl Benzoate] | 2.00 |

| ***Phase D*** | |
|---|---|
| H₂O | |
| Sodium hydroxide 30% | 3.00 |

| ***Phase E*** | |
|---|---|
| **VENUCEANE**™ [*Thermus Thermophilus* Ferment (and) Glycerin] | 5.00 |

| ***Phase F*** | |
|---|---|
| Fragrance | |

Protocol: Weigh phase A.Weigh and mix phase B. Pour phase B into phase A under stirring for 1 hour. Heat phase A+B at 75°C using a water bath. Weigh and mix phase C. Heat phase C at 75°C in a water bath until completely dissolved. Add phase C to phase A+B under stirring. Add phase D under stirring. Add phase E below 35°C and mix well. Add phase F and mix well.

### Examples of ingredients that can be added to this formulation:

LUMISKIN™: active ingredient marketed by SEDERMA (WO2004/024695), which lightens the complexion. It comprises diacetyl Boldine (DAB) in a C8-C10 triglyceride solution.

### 5) After sun cream form

| **Ingredients** | **Weight %** |
|---|---|
| ***Phase A*** | |
| H₂O | Qsp 100 |
| Sodium benzoate | Qs |
| Potassium sorbate | Qs |

| ***Phase B*** | |
|---|---|
| Glycerin | 3.00 |
| Guar gum | 0.15 |
| Xanthan gum | 0.50 |
| Crodesta F-50-PW-(RB) [Sucrose Distearate] | 0.90 |
| Crodesta F-160-PW-(RB) [Sucrose Stearate] | 0.30 |

| ***Phase C*** | |
|---|---|
| Crodamol SS-PA-(RB) [Cetyl Esters] | 1.00 |
| NG INSAPONIFIABLES DE BEURRE DE KARITÉ™ [Butyrospermum | |
| Parkii (Shea Butter) Unsaponifiables] | 0.50 |
| Pripure 3759 [Squalane] | 1.00 |
| Sunflower oil organic [*Helianthus Annuus* Seed Oil] | 7.00 |
| Tocopherol | 0.20 |

| ***Phase D*** | |
|---|---|
| Organique alcohol | 3.00 |

| ***Phase E*** | |
|---|---|
| H₂O | 0.10 |
| Lactic acid | 0.10 |

| ***Phase F*** | |
|---|---|
| **VENUCEANE**™ [*Thermus Thermophilus* Ferment (and) Glycerin] | 3.00 |

| ***Phase G*** | |
|---|---|
| Fragrance | 0.30 |

Protocol: Weigh and mix phase A under stirring. Weigh and mix phase B. Slowly add phase B to phase A while stirring. Homogenized for 30 min. Heat phase A+B at 75°C using a water bath. Weigh and heat phase C at 75°C using a water bath. Add phase C to phase A+B under stirring. Cool while stirring. Add phase D and homogenize under stirring below 40°C. Adjust pH to 5.0-5.5 with phase E at 35°C. Mix well. Add successively phase F and phase G. Mix thoroughly.

NG UNSAPONIFIABLE SHEA BUTTER™: active ingredient marketed by Sederma with nourishing and protective properties for the treatment of skin damaged by the environment.

### 6) Composition for soaking a wipe

| **Ingredients** | **Weight %** |
|---|---|
| ***Phase A*** | |
| H₂O | Qs 100 |
| Carbomer | 0.3 |
| Potassium sorbate | 0.15 |

| ***Phase B*** | |
|---|---|
| Butylen glycol | 4.00 |
| Mixed Parabens | 0.20 |

| ***Phase C*** | |
|---|---|
| Crodasinic LS30 [Sodium Lauroyl Sarcosinate] | 1.00 |
| Crodacol CS90-PA-(RB) [Cetearyl Alcohol] | 1.00 |
| Span 60-PA-(SG) [Polysorbate 60] | 3.00 |
| Tween 60-SS-(RB) [Sorbitan Stearate] | 1.20 |
| Mineral Oil | 10.00 |

| ***Phase D*** | |
|---|---|
| H₂O | 3.00 |
| Sodium hydroxyde | 0.30 |

| ***Phase E*** | |
|---|---|
| **VENUCEANE**™ [*Thermus Thermophilus* Ferment (and) Glycerin] | 4.00 |

| ***Phase F*** | |
|---|---|
| Fragrance | 0.10 |

Protocol: Weigh phase A and let swell 20 minutes. Heat phase B at around 60°C to dissolve the conservatives. Add phase B to phase A and heat at 75°C using a water bath under stirring. Add phase C to phase A+B under stirring. Adjust pH to 6.5 with phase D. Add phase E at 40°C and phase F at 35°C under stirring.

### 7) Spray form

| **Ingredients** | **Weigh %** |
|---|---|
| ***Phase A*** | |
| Sodium hydroxyde | 0.20 |
| Potassium sorbate | 0.10 |
| H₂O | qsp 100 |

| ***Phase B*** | |
|---|---|
| Brij CS20-PA-(RB) [Ceteareth-20] | 1.50 |
| Crodafos CS20A-PA-(MH) [Cetearyl Alcohol (and) Ceteth-20 Phosphate (and) Dicetyl Phosphate] | 3.00 |
| Crodamol GTCC-LQ-(MV) [Caprylic/Capric Trygliceride] | 5.00 |
| Cromollient DP3A-LQ-(MH) [Di-PPG-3 Myristyl Ether Adipate] | 3.00 |
| Cyclohexasiloxane & Cyclopentasiloxane | 1.00 |

| ***Phase C*** | |
|---|---|
| Glycerin | 5.00 |
| Mixed Parabens | 0.20 |

| ***Phase D*** | |
|---|---|
| **VENUCEANE**™ [*Thermus Thermophilus* Ferment (and) Glycerin] | 3.00 |

| ***Phase E*** | |
|---|---|
| Fragrance | 0.10 |

Protocol: Heat phase A at 75°C using a water bath. Melt phase C until dissolved and add to phase A+B at 75°C using a water bath. Mix well. Pour phase A into phase B+C under stirring. Mix thoroughly. Add phases D and E in phase A+B+C at about 45°C. Mix well while cooling.

### 8) Spray form for hair protection

| **Ingredients** | **Weight %** |
|---|---|
| ***Phase A*** | |
| H₂O | Qs 100 |
| Citric acid | 0.24 |
| Disodic phosphate | 1.10 |
| Guar hydroxypropyl | 0.15 |

| ***Phase B*** | |
|---|---|
| Ethanol | 10.00 |
| Incroquat CTC30 (Cetrimonium chloride) | 1.00 |

| ***Phase C*** | |
|---|---|
| **VENUCEANE**™ [*Thermus Thermophilus* Ferment (and) Glycerin] | 1.00 |

| ***Phase D*** | |
|---|---|
| Fragrance | 0.10 |
| Tween 20 (Polysorbate 20) | 0.40 |

Protocol: Disperse phase A until dissolved. Mix phase B and then add it to phase A. Add phase C to phase A+B. Combine phase D and add it to phase A+B+C. Mix well. pH = 6.80.

## Claims

1. *Thermus thermophilus* fermentation medium for use in treatment of infra-red radiation (IR) related damages of the skin and its appendages.

2. Medium for use according to claim 1, **characterized in that** it is used for a treatment to limit the increase of pro-inflammatory mediators (PGE2, IL-6 and IL-8) due to IR.

3. Medium for use according to claim 1 or 2, **characterized in that** it is used for a treatment to limit the decrease of the synthesis of extracellular matrix structuring macromolecules (collagen-1, fibrilin-land/or HSP-47 (heat shock protein)) due to IR.

4. Medium for use according to anyone of the preceding claims, **characterized in that** it is used for a treatment to limit the increase of the formation of reactive oxygen species (ROS) due to IR.

5. Medium for use according to anyone of the preceding claims, **characterized in that** it is used for a treatment to increase ATP synthesis.

6. Medium for use according to anyone of the preceding claims, **characterized in that** it is used for a treatment to improve the quality of skin (stimulation of the synthesis of involucrin, ceramides and/or intercellular lipids).

7. Medium for use according to anyone of the preceding claim, **characterized in that** it is combined with another active ingredient selected from the active filtering radiations, moisturizers, calming, muscle relaxants, slimming, restructurizing, firming, plumping, tensors, acting on microcirculation, acting on inflamation, on free radicals, vitamins, anti-wrinkle, and/or lightening agents.

8. Medium for use according to the preceding claim, **characterized in that** it is used in combination with a sunscreen.

9. Medium for use according to the preceding claim, **characterized in that** the sunscreen is an inorganic filter.

10. Medium for use according to one of the preceding claims, **characterized in that** it is used in a bound form, incorporated or adsorbed on macro-, micro-, and nanoparticles, or macro-, micro-and nanocapsules, for the treatment of textiles, natural or synthetic fibers, wool, and all materials intended to come into contact with the skin and that can be used in clothing, day or night underwear, handkerchief or tissues to exert its effect through this contact skin/textile and allow continuous topical delivery.

## Patentansprüche

1. *Thermus thermophilus*-Fermentationsmedium zur Verwendung bei der Behandlung von durch Infrarotstrahlung (IR) bedingten Schädigungen der Haut und ihrer Anhangsgebilde.

2. Medium zur Verwendung nach Anspruch 1, **dadurch gekennzeichnet, dass** es für eine Behandlung verwendet wird, um die IR-bedingte Erhöhung entzündungsfördernder Vermittlerstoffe (PGE2, IL-6 und IL-8) zu begrenzen.

3. Medium zur Verwendung nach Anspruch 1 oder 2, **dadurch gekennzeichnet, dass** es für eine Behandlung verwendet wird, um die IR-bedingte Verringerung der Synthese von strukturbildenden Makromolekülen (Collagen-1, Fibrilin-1 und/oder HSP-47 (Hitzeschockprotein)) in der extrazellulären Matrix zu begrenzen.

4. Medium zur Verwendung nach einem der vorhergehenden Ansprüche, **dadurch gekennzeichnet, dass** es für eine Behandlung verwendet wird, um die IR-bedingte Erhöhung der Bildung reaktiver Sauerstoffspezies (ROS) zu begrenzen.

5. Medium zur Verwendung nach einem der vorhergehenden Ansprüche, **dadurch gekennzeichnet, dass** es für eine Behandlung zur Erhöhung der ATP-Synthese verwendet wird.

6. Medium zur Verwendung nach einem der vorhergehenden Ansprüche, **dadurch gekennzeichnet, dass** es für eine Behandlung verwendet wird, um die Qualität der Haut zu verbessern (Anregung der Synthese von Involucrin, Ceramiden und/oder interzellulären Lipiden).

7. Medium zur Verwendung nach einem der vorhergehenden Ansprüche, **dadurch gekennzeichnet, dass** es mit einem anderen aktiven Inhaltsstoff kombiniert wird, der aus strahlungsfilternden Wirkstoffen, Feuchtigkeitsspendern, beruhigenden Stoffen, Muskelrelaxanzien, Schlankmachern, restrukturierenden Stoffen, Straffmachern, aufpolsternden Stoffen, die Spannkraft erhöhenden Stoffen, Stoffen, die auf die Mikrozirkulation, auf ein Entzündungsgeschehen oder auf freie Radikale wirken, Vitaminen, Anti-Falten-Stoffen und/oder aufhellenden Mitteln ausgewählt ist.

8. Medium zur Verwendung nach einem der vorhergehenden Ansprüche, **dadurch gekennzeichnet, dass** es in Kombination mit einem Sonnenschutzmittel verwendet wird.

9. Medium zur Verwendung nach einem der vorhergehenden Ansprüche, **dadurch gekennzeichnet, dass** es sich bei dem Sonnenschutzmittel um einen anorganischen Filter handelt.

10. Medium zur Verwendung nach einem der vorhergehenden Ansprüche, **dadurch gekennzeichnet, dass** es in einer gebundenen Form verwendet, in Makro-, Mikro- und Nanopartikel oder Makro-, Mikro- und Nanokapseln eingebaut oder an Makro-, Mikro- und Nanopartikel bzw. Makro-, Mikro- und Nanokapseln adsorbiert ist, für die Behandlung von Textilien, natürlichen oder synthetischen Fasern, Wolle und allen Materialien, die vorgesehen sind, um in Kontakt mit der Haut zu gelangen, und das in Kleidung, in Unterwäsche für tagsüber und nachts, in Taschentüchern oder Papiertüchern verwendet werden kann, um seine Wirkung durch diesen Kontakt zwischen Haut und Textilie auszuüben und eine kontinuierliche topische Abgabe zu ermöglichen.

## Revendications

1. Milieu de fermentation de *thermus thermophilus* pour une utilisation dans un traitement des dommages liés aux radiations infra-rouges (IR) de la peau et de ses phanères.

2. Milieu pour une utilisation selon la revendication 1, **caractérisé en ce qu'**il est utilisé pour un traitement destiné à limiter l'augmentation de médiateurs pro-inflammatoires (PGE2, IL-6 et IL-8) due aux IR.

3. Milieu pour une utilisation selon la revendication 1 ou 2, **caractérisé en ce qu'**il est utilisé pour un traitement destiné à limiter la baisse de la synthèse de macromolécules structurant la matrice extracellulaire (collagène-1, fibriline-1 et/ou HSP-47 (Heat Shock Protein)) due aux IR.

4. Milieu pour une utilisation selon l'une quelconque des revendications précédentes, **caractérisé en ce qu'**il est utilisé pour limiter l'augmentation de la formation d'espèces radicalaires oxygénées (ERO) due aux IR.

5. Milieu pour une utilisation selon l'une quelconque des revendications précédentes, **caractérisé en ce qu'**il est utilisé pour augmenter la synthèse d'ATP.

6. Milieu pour une utilisation selon l'une quelconque des revendications précédentes, **caractérisé en ce qu'**il est utilisé pour améliorer la qualité de la peau (stimulation de la synthèse d'involucrine, de céramides et/ou de lipides intercellulaires).

7. Milieu pour une utilisation selon l'une quelconque des revendications précédentes, **caractérisé en ce qu'**il est associé à un autre ingrédient actif choisi les actifs filtrant les radiations, hydratants, calmants, myorelaxants, amincissants, restructurants, raffermissants, repulpants, tenseurs, agissant sur la microcirculation, agissant sur l'inflammation, sur les radicaux libres, les vitamines, les anti-rides, et/ou éclaircissants.

8. Milieu pour une utilisation selon la revendication précédente, **caractérisé en ce qu'**il est utilisé en combinaison avec un filtre solaire.

9. Milieu pour une utilisation selon la revendication précédente, **caractérisé en ce que** le filtre solaire est un filtre inorganique.

10. Milieu pour une utilisation selon l'une des revendications précédentes, **caractérisé en ce qu'**il est utilisé sous une forme liée, incorporée ou adsorbée sur des macro-, micro-, et nanoparticules, ou sur macro-, micro- et nanocapsules, pour le traitement des textiles, des fibres naturelles ou synthétiques, des laines, et tous matériaux destinés à entrer en contact avec la peau et qui peuvent être employés dans l'habillement, les sous-vêtements de jour ou de nuit, les mouchoirs, ou les tissus, afin d'exercer son effet par l'intermédiaire de ce contact peau/textile et permettre une délivrance topique continue.
